# EUROPÄISCHE PATENTANMELDUNG

(11) **EP 2 982 880 A2**
(43) Veröffentlichungstag der Anmeldung: **10.02.2016**
(21) Anmeldenummer: 15171091.0
(22) Anmeldetag: 09.06.2015
(51) Int. Cl.: F16D 41/10, A61G 13/12

(54) **VORRICHTUNG ZUM SELBSTHEMMENDEN BIDIREKTIONALEN ANTRIEB EINER MEDIZINISCHEN BEHANDLUNGSVORRICHTUNG**

(30) Priorität: 23.06.2014 DE 102014108745
(71) Anmelder: Maquet GmbH, 76437 Rastatt (DE)
(72) Erfinder: Wyslucha, Ulrich, 76356 Weingarten (DE); Biel, Dominik, 76456 Kuppenheim (DE); Obert, Mike, 76593 Gernsbach (DE)
(74) Vertreter: Zacco GmbH

(57) **Zusammenfassung**

Die Erfindung betrifft eine Vorrichtung zum selbsthemmenden bidirektionalen Antrieb einer medizinischen Behandlungsvorrichtung (18) mit einer ersten Sperreinheit zum Sperren der Drehung eines Innenteils (72) in einer ersten Drehrichtung (R1) und zum Freigeben der Drehung des Innenteils (72) in einer zweiten Drehrichtung (R2) und mit einer zweiten Sperreinheit zum Sperren der Drehung des Innenteils (72) in der zweiten Drehrichtung (R2) und zum Freigeben der Drehung des Innenteils (72) in der ersten Drehrichtung (R1). Das Innenteil (72) ist über eine Abtriebswelle (55) mit der medizinischen Behandlungsvorrichtung (18) verbunden. Es sind zwei mit Hilfe eines Antriebselements (36) in jeweils eine Freigabeposition bewegbare Freigabeelemente (122a bis 122c, 123a bis 123c) vorgesehen, die das Sperren der Drehung des Innenteils (72) in jeweils eine Drehrichtung (R1, R2) verhindern, so dass eine Drehung des Innenteils (72) und der über die Abtriebswelle (55) mit dem Innenteil (72) verbundenen medizinischen Behandlungsvorrichtung (18) nur durch das Antriebselement (36) möglich ist.

## Beschreibung

Die Erfindung betrifft eine Vorrichtung zum selbsthemmenden bidirektionalem Antrieb einer medizinischen Behandlungsvorrichtung, mit einem um eine Drehachse drehbaren Innenteil, einem rotationsfesten Außenteil mit einer kreisförmigen Öffnung, die das Innenteil umgibt und konzentrisch um die Drehachse angeordnet ist, einer um die Drehachse drehbaren Abtriebswelle, die mit dem Innenteil und mit der Behandlungsvorrichtung verbindbar ist und mit einem Antriebselement zum Drehen des Innenteils zusammen mit der Abtriebswelle. Ferner umfasst die Vorrichtung einen ersten Klemmkörper und einen zweiten Klemmkörper, die in einem Zwischenraum zwischen dem Innenteil und dem Außenteil angeordnet sind.

Bei der Behandlung von Patienten ist es oft erforderlich, dass Teile des Patienten, insbesondere Gliedmaßen fixiert und gezielt bewegt werden müssen. Insbesondere bei Operationen, bei denen die Lage eines Körperteils mehrfach gezielt geändert werden muss, sind einstellbare Behandlungsvorrichtungen erforderlich. So muss ein Patientenbein bei einer hüftprothetischen Prozedur nach dem "direct interior approach" sowie bei der "total hip arthroplasty" wiederholt rotiert werden. Das Patientenbein ist hierbei in einer Traktions- bzw. Zugeinheit freitragend gerade eingespannt. Solche Traktions- bzw. Zugeinheiten werden in Verbindung mit Operationstischen auch als Extensionsvorrichtungen bezeichnet. Dabei muss der Rotationswinkel des Beins entsprechend dem chirurgischen Workflow und den medizinischen Notwendigkeiten mehrfach geändert werden. Der maximale übliche Rotationsbereich beträgt 180°.

Zum Ändern des Rotationswinkels eines in einer Extensionsvorrichtung eingespannten Patientenbeins muss ein Bediener eine Klemmschraube zur Freigabe der Rotation öffnen, sodass die Rotation des Patientenbeins durch die Extensionsvorrichtung freigegeben ist. Anschließend dreht der Operateur das Patientenbein in die gewünschte Position. Dabei kann die Fußspitze des Patienten als Indikator für den Drehwinkel genutzt werden. Das Patientenbein muss dann im gewünschten Rotationswinkel manuell gehalten werden, wobei durch die anatomische Struktur des Beins eine Rückstellkraft in einem von dem gewünschten Rotationswinkel abweichenden Rotationswinkel auftritt. Anschließend wird die Klemmschraube angezogen, sodass das Patientenbein von der Extensionsvorrichtung in dem gewünschten Rotationswinkel fixiert wird. Um das Patientenbein in die vorherige Lage zurückzubringen oder in einen anderen Rotationswinkel zu bringen, muss die Klemmschraube wieder geöffnet werden, das Patientenbein wieder kontrolliert zurück oder in die gewünschte Lage gedreht werden. Anschließend wird die Klemmschraube wieder fixiert.

Abhängig vom chirurgischen Workflow muss diese Prozedur während einer Operation mehrfach wiederholt werden. Im erläuterten Ablauf sind die Tätigkeiten Lösen/Festziehen der Klemmschraube und Rotieren/Halten des Patientenbeins in der gewünschten Position teilweise gleichzeitig durchzuführen. Das Rückstellbestreben des Patientenbeins in eine definierte Lage ist dabei oft erschwerend und führt zu einer erheblichen Belastung des Operateurs, da erhebliche Kraft und Konzentration zur Koordination der erforderlichen Arbeitsschritte benötigt werden. Eine feinfühlige Positionierung des Patientenbeins ist damit während einer Operation nur eingeschränkt möglich, sodass hierdurch ein Risiko für den Patienten im Operationsablauf gegeben ist. Um dieses Risiko in Grenzen zu halten, ist es üblich, dass eine Person die Klemmschraube öffnet und schließt und eine zweite Person das Patientenbein in die gewünschte Lage bringt und dort hält, bis die Klemmschraube wieder festgestellt ist. Mit zwei Personen ist der Aufwand hierfür relativ hoch.

Aufgabe der Erfindung ist es, eine Vorrichtung anzugeben, mit der die Position einer medizinischen Behandlungsvorrichtung einfach und sicher geändert werden kann.

Diese Aufgabe wird durch eine Vorrichtung zum selbsthemmenden bidirektionalen Antrieb einer medizinischen Behandlungsvorrichtung mit den Merkmalen des Anspruchs 1 gelöst. Vorteilhafte Weiterbildungen der Erfindung sind in den abhängigen Ansprüchen angegeben.

Durch die in entgegengesetzte Drehrichtungen wirkenden Sperreinheiten zum Sperren der Drehung des Innenteils wird eine Drehung der Abtriebswelle und somit auch eine Drehung einer mit der Abtriebswelle verbundenen Behandlungsvorrichtung, wie einer Fußaufnahme zur Aufnahme des Fußes eines Patientenbeins verhindert, wenn ein abtriebsseitiges Drehmoment anliegt. Eine Drehung des Innenteils und somit der Abtriebswelle ist durch die erfindungsgemäße Vorrichtung zum selbsthemmenden bidirektionalen Antrieb einer medizinischen Behandlungsvorrichtung nur durch das Antriebselement möglich, da dieses das erste Freigabeelement zur Freigabe der ersten Drehrichtung sowie das zweite Freigabeelement zur Freigabe der zweiten Drehrichtung bewegt. Dadurch ist einfach eine Drehung der Abtriebswelle und somit eine Verstellung der medizinischen Behandlungsvorrichtung durch Betätigen des Antriebselements möglich, wodurch eine einfache Handhabung der medizinischen Behandlungsvorrichtung, insbesondere eine Bedienung durch nur eine Person, einfach möglich ist. Insbesondere kann auf separat zu betätigende Klemm- oder Feststellelemente verzichtet werden, da eine Feststellung automatisch über die in entgegengesetzte Drehrichtungen wirkenden Sperreinheiten sichergestellt ist. Auch ist über das Antriebselement eine exakte Drehung der Abtriebswelle einfach möglich, sodass sich gewünschte Winkelpositionen der Abtriebswelle und der mit der Abtriebswelle verbundenen Behandlungsvorrichtung einfach einstellen lassen.

Besonders vorteilhaft ist, wenn zusätzlich ein Drehspindelantrieb vorgesehen ist, durch den die medizinische Behandlungsvorrichtung in Richtung der Drehachse oder parallel zur Drehachse verschiebbar ist. Dadurch kann die medizinische Behandlungsvorrichtung entlang der Drehachse bzw. parallel zur Drehachse längs verschoben und zusätzlich der Rotationswinkel der Behandlungsvorrichtung mit Hilfe des Antriebselements exakt eingestellt werden, um z. B. eine Zugkraft auf das Patientenbein auszuüben. Die Drehspindel des Drehspindelantriebs ist vorzugsweise durch die Abtriebswelle mittig hindurchgeführt, wobei die Drehspindel frei drehbar zur Abtriebswelle angeordnet ist.

Besonders vorteilhaft ist es, wenn das Antriebselement bei einer Drehung in die erste Drehrichtung zuerst das erste Freigabeelement aus einer ersten Neutralposition in die erste Freigabeposition bewegt, in der das erste Freigabeelement das Sperren der Drehung des Innenteils in der ersten Drehrichtung durch die erste Sperreinheit verhindert, und bei einer weiteren Drehung das Innenteil in die erste Drehrichtung dreht. Ferner bewegt das Antriebselement bei einer Drehung in die zweite Drehrichtung zuerst das zweite Freigabeelement aus einer zweiten Neutralposition in die zweite Freigabeposition, in der das zweite Freigabeelement das Sperren der Drehung des Innenteils in der zweiten Drehrichtung durch die zweite Sperreinheit verhindert, und bei einer weiteren Drehung in der zweiten Drehrichtung das Innenteil in die zweite Drehrichtung dreht. Somit erfolgt bei einer Drehung des Antriebselements in die erste Drehrichtung zuerst eine Freigabe der Drehung des Innenteils in der ersten Drehrichtung bzw. das Aufheben des Sperrens der Drehung des Innenteils in der ersten Drehrichtung und anschließend die Drehung des Innenteils und der mit dem Innenteil gekoppelten Abtriebswelle. In gleicher Weise erfolgt bei einer Drehung des Antriebselements in die zweite Drehrichtung zuerst eine Freigabe der Drehung des Innenteils in der zweiten Drehrichtung und bei einer weiteren Drehbewegung des Antriebselements in der zweiten Drehrichtung eine Drehung des Innenteils in die zweite Drehrichtung. Vorzugsweise werden das erste Freigabeelement und das zweite Freigabeelement aus der ersten Freigabeposition bzw. zweiten Freigabeposition automatisch zurück in die erste bzw. zweite Neutralposition, vorzugsweise durch eine Federkraft aus der jeweiligen Freigabeposition in die Neutralposition, bewegt. Dadurch ist eine einfache Handhabung der Vorrichtung ohne Umschaltung der gewünschten Drehrichtung oder Entriegelung möglich.

Ferner ist es vorteilhaft, wenn der erste Klemmkörper in einem zwischen dem Außenteil und dem Innenteil gebildeten ersten Klemmbereich bei einer Drehung des Innenteils in die erste Drehrichtung einklemmbar ist, und wenn der zweite Klemmkörper in einen zwischen dem Außenteil und dem Innenteil gebildeten zweiten Klemmbereich bei einer Drehung des Innenteils in die zweite Drehrichtung einklemmbar ist. Das Einklemmen des ersten Klemmkörpers im ersten Klemmbereich bzw. des zweiten Klemmkörpers im zweiten Klemmbereich erfolgt nur dann, wenn sich das erste Freigabeelement bzw. das zweite Freigabeelement nicht in ihrer Freigabeposition befinden. Durch das Verklemmen der Klemmkörper im zweiten Klemmbereich wird sichergestellt, dass eine Sperrung der Drehbewegung in die erste Drehrichtung und in die zweite Drehrichtung nur dann erfolgt, wenn die Freigabeelemente nicht mit Hilfe der Antriebseinheit in ihre Freigabeposition bewegt worden sind. Somit wird eine Drehung der Abtriebswelle über die medizinische Behandlungsvorrichtung einfach und wirkungsvoll vermieden, ohne dass hierfür bestimmte Bedienhandlungen, wie das Feststellen von Klemmschrauben oder Ähnliches, erforderlich sind. Solche Klemmanordnungen mit Klemmkörpern, die zwischen einem Außenteil und einem Innenteil angeordnet sind und die in einem zwischen Außenteil und Innenteil gebildeten Klemmbereich eingeklemmt werden, sind beispielsweise aus sogenannten Klemmkörperfreiläufen bekannt, die eine freie Drehung eines Innenteils und zu einem Außenteil in einer ersten Drehrichtung ermöglichen und die eine Verbindung zwischen Innen- und Außenteil bei den Drehungen die entgegengesetzte Drehrichtung herstellen.

Durch das Klemmen der Klemmkörper im entsprechenden Klemmbereich ist eine einfache und nahezu wartungsfreie Anordnung gegeben, die eine ungewünschte Rotation der medizinischen Behandlungsvorrichtung um die Drehachse sicher verhindert.

Weiterhin ist es vorteilhaft, wenn ein elastisch verformbares Element zwischen dem ersten Klemmkörper und dem zweiten Klemmkörper angeordnet ist, das den ersten Klemmkörper in den ersten Klemmbereich und den zweiten Klemmkörper in den ersten Klemmbereich drückt. Dadurch sind die Klemmkörper sicher im jeweiligen Klemmbereich angeordnet, sodass eine spielfreie Sperrung der Drehbewegung möglich ist und dadurch eine dauerhafte exakte Positionierung der medizinischen Behandlungsvorrichtung erreicht wird.

Besonders vorteilhaft ist es, wenn das elastische Element eine Feder, vorzugsweise eine zwischen den Klemmkörpern angeordnete Spiralfeder, ist. Alternativ kann das elastisch verformbare Element durch einen Elastomerblock oder Polymerblock gebildet sein. Durch diese Elemente kann auf einfache Weise sichergestellt werden, dass die Klemmkörper in ihren jeweiligen Klemmbereich gedrückt und dort sicher gehalten werden. Ferner kann der jeweilige Klemmkörper bei der Bewegung des jeweiligen Freigabeelements in die Freigabeposition gegen die Federkraft aus dem Klemmbereich gedrückt werden, sodass die Bewegung durch den jeweiligen Klemmkörper nicht mehr gesperrt ist.

Ferner ist es vorteilhaft, wenn das erste Freigabeelement auf der dem elastisch verformbaren Element abgewandten Seite des ersten Klemmkörpers und das zweite

Freigabeelement auf der dem elastisch verformbaren Element abgewandten Seite des zweiten Klemmkörpers angeordnet sind. Dadurch ist eine einfache und kompakte Anordnung der Klemmkörper und der Freigabeelemente möglich.

Ferner ist es vorteilhaft, wenn die erste Sperreinheit und die zweite Sperreinheit bei einem an der Abtriebswelle und/oder an dem Innenteil anliegenden abtriebsseitigen Drehmoment eine Drehung des Innenteils und der Abtriebswelle ohne Betätigung des Antriebselements verhindern. Dadurch ist eine einfachere und sichere Handhabung der Behandlungsvorrichtung ermöglicht, da auch bei einem an dem Innenteil anliegenden abtriebsseitigen Drehmoment eine Drehung des Innenteils und der Abtriebswelle ohne Betätigung des Antriebselements sicher verhindert wird. Somit ist eine einfachere und sichere Handhabung der Behandlungsvorrichtung möglich.

Ferner ist es vorteilhaft, wenn die Vorrichtung eine Drehung der Abtriebswelle nur durch Betätigen des Antriebselements ermöglicht. Damit ist sichergestellt, dass eine Drehung der medizinischen Behandlungsvorrichtung durch ein abtriebsseitiges Drehmoment sicher verhindert und somit eine sichere Handhabung der medizinischen Behandlungsvorrichtung möglich ist.

Ferner ist es vorteilhaft, wenn das Antriebselement über mindestens ein Eingriffselement mit dem Innenteil in Eingriff steht, wobei das Eingriffselement in einer Aussparung des Innenteils mit Spiel aufgenommen ist und/oder wobei das Eingriffselement in einer Aussparung des Antriebselements mit Spiel aufgenommen ist. Das Spiel hat vorzugsweise einen Wert im Bereich von 0,5 mm bis 5 mm oder von 0,01° bis 2°.

Konkret kann das Eingriffselement als Stift und die Aussparung als Bohrung ausgeführt sein, wobei der Stift in die Bohrung ragt und der Durchmesser der Bohrung vorzugsweise um einen Wert im Bereich von 0,5 mm bis 5 mm größer als der Durchmesser des Stifts ist.

Das Antriebselement ist dabei vorzugsweise direkt, d. h. ohne Spiel, mit den Freigabeelementen gekoppelt bzw. steht direkt ohne Spiel mit diesen in Eingriff. Dadurch kann auf einfache Weise sichergestellt werden, dass zuerst das erste Freigabeelement in die erste Freigabeposition gebracht wird, sodass das Sperren der ersten Sperreinheit verhindert wird, und dass anschließend eine Drehung des Innenteils in die erste Drehrichtung erfolgt. In gleicher Weise wird bei einer Drehung der Antriebseinheit in die zweite Drehrichtung zuerst das zweite Freigabeelement in die zweite Freigabeposition bewegt, in der das zweite Freigabeelement ein Klemmen des zweiten Klemmkörpers im zweiten Klemmbereich verhindert, und anschließend erfolgt eine Drehung des Innenteils durch die Antriebseinheit in der zweiten Drehrichtung. Dadurch ist weder eine Drehrichtungsumschaltung noch eine zusätzliche Arretierung erforderlich. Es muss lediglich ein Antrieb mit Hilfe des Antriebselements erfolgen, um die gewünschte Drehbewegung in einer Bedienhandlung freizugeben und auszuführen.

Besonders vorteilhaft ist es, wenn das erste Freigabeelement und das zweite Freigabeelement mit einem Betätigungselement in Wirkverbindung stehen, und wenn das Betätigungselement bei einer Betätigung das erste Freigabeelement in die erste Freigabeposition und das zweite Freigabeelement in die zweite Freigabeposition bewegt. Dadurch kann die Sperrung durch die erste Sperreinheit und die Sperrung durch die zweite Sperreinheit gleichzeitig unabhängig von der Betätigung des Antriebselements aufgehoben werden, sodass dann eine Drehung der Abtriebswelle und der mit der Abtriebswelle verbundenen medizinischen Behandlungsvorrichtung auch abtriebsseitig erfolgen kann. Somit erfolgt eine Freigabe der Rotation durch Betätigung des Betätigungselements. Durch die Freigabe der Rotation kann die medizinische Behandlungsvorrichtung frei gedreht werden, beispielsweise um die Funktion und Beweglichkeit von Gelenken einer mit der Behandlungsvorrichtung verbundenen Gliedmaße eines Patienten zu beurteilen, wie beispielsweise die Drehung eines Patientenbeins nach dem Einsetzen einer Hüftendoprothese oder eines künstlichen Kniegelenks.

Weiterhin ist es vorteilhaft, wenn eine erste Sperr- und Freigabeanordnung zumindest die erste Sperreinheit, die zweite Sperreinheit, die erste Freigabeeinheit und die zweite Freigabeeinheit umfasst und wenn die Vorrichtung mindestens eine zweite Sperr- und Freigabeanordnung umfasst, wobei der Aufbau und die Funktion der zweiten Sperr- und Freigabeanordnung mit dem Aufbauen der ersten Sperr- und Freigabeanordnung übereinstimmen. Somit kann die zweite Sperr- und Freigabeanordnung zumindest eine dritte Sperreinheit, eine vierte Sperreinheit, eine dritte Freigabeeinheit und eine vierte Freigabeeinheit umfassen. Die mindestens zwei Sperr- und Freigabeanordnungen sind in gleichen Winkelabständen um die Drehachse herum angeordnet. Dadurch werden die zwischen Außenteil und Innenteil zum Sperren der Bewegung auftretenden Kräfte gleichmäßig auf den Umfang des Innenteils und Außenteils verteilt, sodass die auf das Innenteil durch die Sperr- und Freigabeanordnungen orthogonal zur Drehachse wirkende Kräfte in der Summe vorzugsweise Null sind. Dadurch müssen weder das Innenteil noch die Abtriebswelle Querkräfte durch die Sperr- und Freigabeanordnungen aufnehmen, wo ein einfacher und kompakter Aufbau möglich ist Besonders vorteilhaft ist es, wenn die Vorrichtung drei Sperr- und Freianordnungen umfasst, deren Aufbau und Funktion übereinstimmen und die in gleichen Winkelabständen um die Drehachse herum angeordnet sind. Dadurch ist ein besonders einfacher und kompakter Aufbau möglich.

Besonders vorteilhaft ist es, wenn die erste Sperr- und Freigabeanordnung zumindest das erste Freigabeelement, das zweite Freigabeelement, den ersten Klemmkörper und den zweiten Klemmkörper umfasst, wenn die zweite Sperr- und Freigabeanordnung zumindest ein drittes Freigabeelement, ein viertes Freigabeelement, einen dritten Klemmkörper und einen vierten Klemmkörper umfasst, wenn die dritte Sperr-und Freigabeanordnung zumindest ein fünftes Freigabeelement, ein sechstes Freigabeelement, einen fünften Klemmkörper und einen sechsten Klemmkörper umfasst, wenn das erste, dritte und fünfte Freigabeelement derart miteinander verbunden sind, dass sie gemeinsam um die Drehachse bewegbar sind, und wenn das zweite, vierte und sechste Freigabelelement derart miteinander verbunden sind, dass sie gemeinsam um die Drehachse bewegbar sind. Das erste, dritte und fünfte Freigabeelement sind gemeinsam relativ zum zweiten, vierten und sechsten Freigabeelement bewegbar, vorzugsweise um einen Winkel im Bereich von 0,5 ° bis 5°. Dadurch ist eine gemeinsame Bewegung des ersten, dritten und fünften Freigabeelements relativ zum zweiten, vierten und sechsten Freigabeelement möglich, sodass eine solche Bewegung mit Hilfe des Betätigungselements erzeugt werden kann, wodurch alle Freigabeelemente gleichzeitig in ihre jeweilige Freigabeposition bewegt werden können und ein Klemmen des jeweiligen Klemmkörpers im jeweiligen Klemmbereich einfach verhindert wird. Dadurch kann die komplette Aufhebung der Sperrwirkung der ersten Sperreinheit und der zweiten Sperreinheit bzw. der entsprechenden Sperreinheiten der weiteren Sperr- und Freigabeanordnungen auf einfache Art und Weise durch ein Betätigen des Betätigungselements erreicht werden.

Ferner ist es vorteilhaft, wenn das Betätigungselement beim Betätigen das erste, dritte und fünfte Freigabeelement gemeinsam in die erste Drehrichtung und das zweite, vierte und sechste Freigabeelement in die zweite Drehrichtung, derart bewegt, dass das erste Freigabeelement den ersten Klemmkörper kontaktiert und ein Klemmen des ersten Klemmkörpers im ersten Klemmbereich verhindert, dass das zweite Freigabeelement den zweiten Klemmkörper kontaktiert und ein Klemmen des zweiten Klemmkörpers im zweiten Klemmbereich verhindert, dass das dritte Freigabeelement einen dritten Klemmkörper der zweiten Sperr- und Freigabeanordnung kontaktiert und ein Klemmen des dritten Klemmkörpers in einem zwischen dem Innenteil und dem Außenteil gebildeten dritten Klemmbereich verhindert, dass das vierte Freigabeelement einen vierten Klemmkörper der zweiten Sperr- und Freigabeanordnung kontaktiert und ein Klemmen des vierten Klemmkörpers in einem zwischen dem Innenteil und dem Außenteil gebildeten vierten Klemmbereichs verhindert, dass das fünfte Freigabeelement einen fünften Klemmkörper der dritten Sperr-und Freigabeanordnung kontaktiert und ein Klemmen des fünften Klemmkörpers in einem zwischen dem Innenteil und dem Außenteil gebildeten fünften Klemmbereich verhindert, und/oder dass das sechste Freigabeelement einen sechsten Klemmkörper der dritten Sperr- und Freigabeanordnung kontaktiert und ein Klemmen des sechsten Klemmkörpers in einem zwischen dem Innenteil und dem Außenteil gebildeten sechsten Klemmbereich verhindert. Dadurch erfolgt eine einfache komplette Freigabe der Drehung, sodass dann eine Drehung auch aufgrund eines abtriebsseitigen Drehmoments möglich ist. Die Sperrwirkung der jeweiligen Sperreinheiten wird durch eine Betätigung des Betätigungselements somit aufgehoben. Das Betätigungselement kann dabei so ausgebildet sein, das es im betätigten Zustand verbleibt, solange es nicht von einer Bedienperson vom betätigten Zustand in den unbetätigten Zustand bewegt wird. Alternativ kann das Betätigungselement durch eine Rückstellkraft vom betätigten Zustand in den nicht betätigten Zustand zurückbewegt werden. In diesem Fall müsste eine Bedienperson das Betätigungselement im betätigten Zustand halten, solange die Rotation der Abtriebswelle freigegeben sein soll. Das Rückstellen kann durch eine Feder erfolgen.

Vorzugsweise sind die Klemmkörper als Klemmrollen ausgebildet, deren Längsachsen parallel zur Drehachse angeordnet sind. Die Querschnittsfläche des Innenteils in einer Ebene quer zur Drehachse ist im Wesentlichen die eines gleichschenkligen Dreiecks.

Das Antriebselement ist vorzugsweise als Handrad ausgebildet, das um die Drehachse herum gedreht wird. Vorzugsweise ist das Handrad direkt mit den Eingriffselementen zum Betätigen der Freigabeelemente und zur Drehung des Innenteils verbunden. Alternativ kann eine Getriebestufe, vorzugsweise mit einer Untersetzung, zwischen dem Handrad und den Eingriffselementen vorgesehen sein.

Im Ergebnis kann der Operateur durch die Erfindung insbesondere ein mit der medizinischen Behandlungsvorrichtung verbundenes Patientenbein mit Hilfe der Antriebseinheit die gewünschte Richtung drehen und eine gewünschte Winkelposition einstellen, wobei gleichzeitig eine Rückstellbewegung des Patientenbeins automatisch durch die Sperreinheiten verhindert wird. Die Sperreinheiten sind entgegengesetzt wirkend. Das Ermöglichen der ungehemmten Rotation der medizinischen Behandlungsvorrichtung durch Betätigen des Betätigungselements ermöglicht insbesondere eine Funktionskontrolle bei Hüftgelenkoperationen, insbesondere eine Funktionskontrolle einer eingesetzten Hüftendoprothese. Die Umschaltung zwischen der gesperrten Drehung mit Hilfe der Sperreinheiten und die Freigabe mit Hilfe des Bedienelements ist intraoperativ von nur einer Bedienperson, insbesondere dem Operateur, problemlos durch Betätigen des Betätigungselements möglich.

Durch die automatische Sperrung einer Rückstellbewegung erfolgt eine spürbare Entlastung des Bedieners, insbesondere des Operateurs. Ferner kann dadurch die Präzision der Positionierung der medizinischen Behandlungsvorrichtung in einer gewünschten Winkelposition erhöht werden. Auch die Korrektur des Rotationswinkels um kleine Winkelbeträge ist einfach durch die stufenlose Verstellung und automatische Sperrung der Drehbewegung einfach möglich. Auch das Risiko von ungewollten Positionsänderungen der medizinischen Behandlungsvorrichtung wird durch die automatische Sperrung der Drehbewegung durch die Sperreinheiten verhindert. Der chirurgische Workflow bei einer Operation oder Behandlung eines Patienten wird vereinfacht und beschleunigt, da weniger Handgriffe als bei bekannten Klemmvorrichtungen zum Einstellen der Rotationen der medizinischen Behandlungsvorrichtung notwendig sind.

Weitere Merkmale und Vorteile der Erfindung ergeben sich aus der nachfolgenden Beschreibung, die die Erfindung anhand von Ausführungsbeispielen im Zusammenhang mit den beigefügten Figuren näher erläutert. Es zeigen:
- Fig. 1: eine perspektivische Darstellung eines Operationstisches mit einem Extensionsset für Hüftarthroskopie und minimal-invasiver Hüftendoprothetik, das eine Einstelleinheit zur Rotations- und Längenverstellung einer Fußaufnahme umfasst;
- Fig. 2: eine perspektivische Seitenansicht der Einstelleinheit;
- Fig. 3: einen Längsschnitt der Einstelleinheit;
- Fig. 4: eine vergrößerte Darstellung eines Ausschnitts des Längsschnitts nach Fig. 3 mit Elementen eines Rotationsantriebs;
- Fig. 5a: eine Seitenansicht einer Anordnung von ausgewählten Elementen von Sperr-und Freigabeeinheiten des Rotationsantriebs nach Fig. 4;
- Fig. 5b: eine Schnittdarstellung der Anordnung nach Fig. 5a entlang der Schnittlinie A-A, wobei zusätzlich ein Außenteil des Rotationsantriebs dargestellt ist;
- Fig. 5c: eine Schnittdarstellung der Anordnung nach Fig. 5a entlang der Schnittlinie B-B;
- Fig. 6: eine perspektivische Darstellung eines Querschnitts des Rotationsantriebs entlang der in Fig. 5a angegebenen Schnittlinie A-A;
- Fig. 7a: eine weitere Ansicht einer Anordnung von Elementen des Rotationsantriebs in einer teilgeschnittenen Darstellung;
- Fig. 7b: ein Querschnitt der Anordnung nach Fig. 7a entlang der Schnittlinie A-A;
- Fig. 8: eine weitere perspektivische Darstellung von Elementen des Rotationsantriebs, in der Elemente zur Rotationsfreigabe gut sichtbar sind; und
- Fig. 9: eine Detaildarstellung von Elementen zur Rotationsfreigabe.

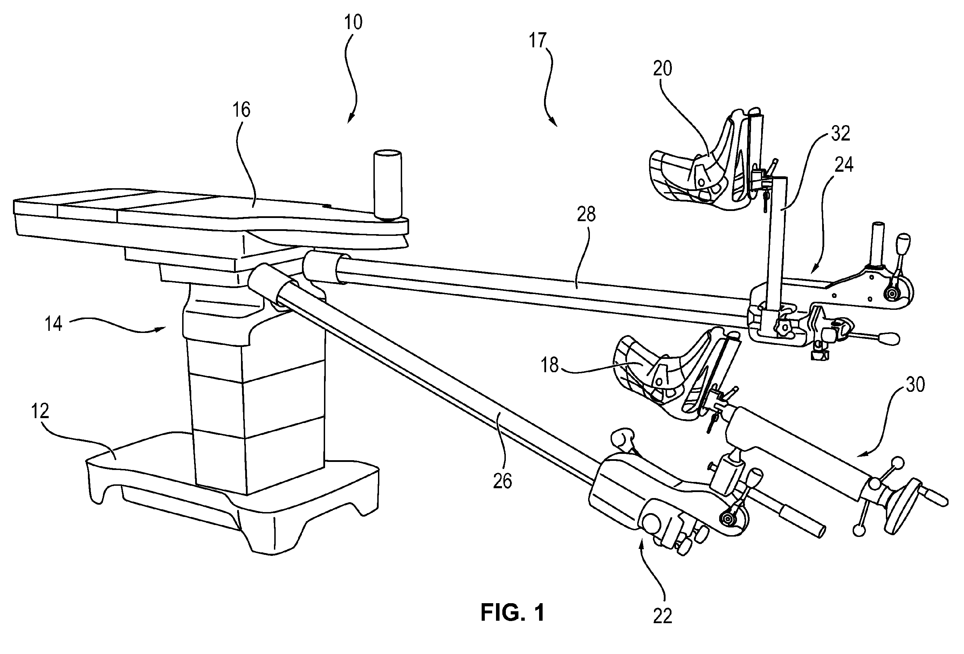

Figur 1 zeigt eine perspektivische Darstellung einer Anordnung eines Operatonstisches 10 mit einem Extensionsset 17, die vorzugsweise zur Hüftarthroskopie und minimal-invasiver Hüftendoprothetik eingesetzt wird. Der Operationstisch 10 hat einen Opertionstischfuß 12, eine Operationstischsäule 14 und eine Patientenlagerfläche 16.

Das Extensionsset 17 umfasst einen ersten Extensionsholm 26 und einen zweiten Extensionsholm 28, die jeweils mit einem ersten Ende mit dem Operationstisch 10 verbunden sind. An dem gegenüberliegenden zweiten Ende des ersten Extensionsholms 26 ist dieser über eine erste Verbindungseinheit 22 und über eine Einstelleinheit 30 zur Rotations- und Längenverstellung mit einer ersten Fußaufnahme 18 verbunden. Der zweite Extensionsholm 28 ist über eine zweite Verbindungseinheit 22 mit einer zweiten Fußaufnahme 20 verbunden.

Die erste Fußaufnahme 18 dient zur Aufnahme des Fußes des zu operierenden Patientenbeins und die zweite Fußaufnahme 20 dient zur Aufnahme des Fußes des nicht zu operierenden Patientenbeins. Somit ist eine Einstelleinheit zur Rotations- und Längenverstellung zwischen der zweiten Verbindungseinheit 24 und der zweiten Fußaufnahme 20 nicht erforderlich. Die zweite Fußaufnahme 20 ist über einen Verbindungsstab 32 mit einer zweiten Verbindungseinheit 24 verbunden. Die Verbindungseinheiten 22, 24 sind auf den jeweiligen Extensionsholmen 26, 28 verschiebbar und feststellbar angeordnet, um das Extensionsset 17 an die Patientenstatur anzupassen. Die Einstelleinheit 30 zur Rotations- und Längenverstellung wird auch als Zugspindelaggregat bezeichnet.

Alternativ kann auch eine Einstelleinheit zwischen der Verbindungseinheit 24 und der Fußaufnahme 20 angeordnet sein, deren Aufbau und Funktion mit dem Aufbau und Funktion der Einstelleinheit 30 übereinstimmt.

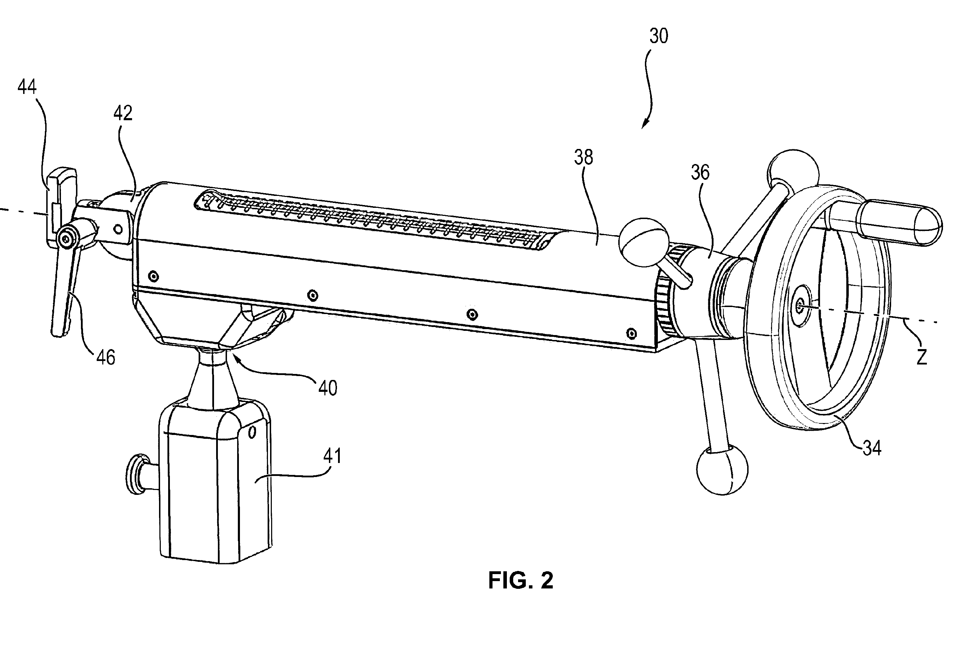

Figur 2 zeigt eine perspektivische Seitenansicht der Einstelleinheit 30. Die Einstelleinheit 30 hat ein erstes Handrad 34 zur axialen Verschiebung eines Verbindungselements 44 entlang einer Längs- und Drehachse Z der Einstelleinheit 30. Das Verbindungselement 44 dient zum Verbinden der Einstelleinheit 30 mit der ersten Fußaufnahme 18. Die Einstelleinheit 30 umfasst ferner ein zweites Handrad 36 zur Rotationseinstellung des Verbindungselements 44, so dass mit Hilfe des Handrads 36 der Drehwinkel der ersten Fußaufnahme 18 änderbar ist.

In einem Gehäuse der Einstelleinheit 30 ist eine mit den Drehrädern 34, 36 in Wirkverbindung stehende Mechanik angeordnet, mit der das Verbindungselement 44 über einen Positionierungskopf 42 verbunden ist. Über den Positionierungskopf 42 ist die Lage des Verbindungselements 44 um eine Drehachse orthogonal zur Längs- und Drehachse Z der Einstelleinheit 30 schwenkbar. Mit Hilfe eines Klemmverschlusses 46 des Positionierungskopfs 42 ist das Verbindungselement 44 in einer Schwenkposition feststellbar. Über ein Gelenk 40 ist die Einstelleinheit 30 mit einer Gelenkkopfhalterung 41 der Verbindungseinheit 22 verbunden.

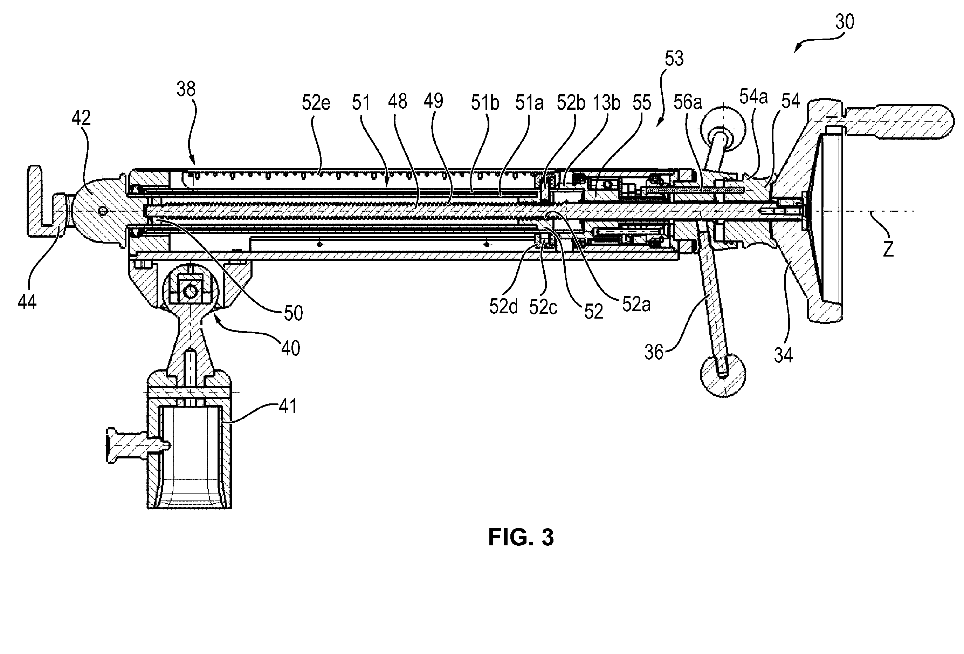

Figur 3 zeigt einen Längsschnitt einer Einstelleinheit 30. Die Einstelleinheit 30 hat eine mit dem ersten Handrad 34 fest verbundene Antriebsspindel 48, die zum Antrieb einer Teleskoprohranordnung 51 zum axialen Verschieben des Positionierungskopfs 42 dient. Die Teleskoprohranordnung 51 ist in den Figuren in einer eingefahrenen Position dargestellt. Die Teleskoprohranordnung 51 hat ein inneres Teleskoprohr 51a und ein äußeres Teleskoprohr 51b. Das äußere Teleskoprohr 51b ist in einer stirnseitigen Öffnung einer Abtriebswelle 55 eines mithilfe des zweiten Handrads 36 angetriebenen Rotationsantriebs 53 drehfest aufgenommen. Der Aufbau und die Funktion des Rotationsantriebs 53 werden nachfolgend in Verbindung mit den weiteren Figuren noch näher erläutert.

Das dem ersten Antriebsrad 34 zugewandte Ende des inneren Teleskoprohrs 51a ist in einem mithilfe der Antriebsspindel 48 bewegbaren Schlitten 52, dessen Innengewinde 52a mit dem Außengewinde 49 der Antriebsspindel 48 in Eingriff steht, fest verbunden. Der Antriebsschlitten 52 hat eine erste nach oben durch einen im äußeren Teleskoprohr 51b vorhandenen oberen Schlitz hindurchragende Nase 52b und eine nach unten durch einen zweiten unteren Schlitz im äußeren Teleskoprohr 51b hindurchragende Nase 52c. Dadurch wird der Schlitten 52 bei einer Drehung des äußeren Teleskoprohrs 51b mit Hilfe des Rotationsantriebs 53 zusammen mit dem äußeren Teleskoprohr 51b gedreht, so dass somit auch das drehfest mit dem Schlitten 52 verbundene innere Teleskoprohr 51a und der Positionierungskopf 42 zusammen mit dem äußeren Teleskoprohr 51b gedreht wird. Beim Ausfahren des inneren Teleskoprohrs 51a aus dem äußeren Teleskoprohr 51b wird der Positionierungskopf 42 somit unter Beibehaltung seiner Winkelstellung ein- und ausgefahren. Das dem Positionierungskopf 42 zugewandte Ende der Antriebsspindel 48 ist in einem Lager 50 drehbar aufgenommen, wobei die Antriebsspindel 48 derart mit dem Lager 50 verbunden ist, dass das Lager 50 beim Ein- und Ausfahren des inneren Teleskoprohrs 51a an dessen Innenwand entlanggleitet. Das äußere Teleskoprohr 51b ist mit dem der Abtriebswelle 55 gegenüberliegenden Ende in einer in dem Gehäuse 38 ausgebildeten Lagerbuchse aufgenommen, so dass dieses Ende des äußeren Teleskoprohrs 51b sicher gehalten und drehbar relativ zum Gehäuse 38 ist. Die Nasen 52b, 52c des Schlittens 52 stehen mit einem umlaufenden Indikatorring 52d im Eingriff, über den die axiale Position des inneren Teleskoprohrs 51a auf einer von außen durch ein Sichtfenster im Gehäuse 38 sichtbaren Skala 52e angezeigt wird. Durch die Drehung der Antriebsspindel 48 mit Hilfe des ersten Handrads 34 kann das innere Teleskoprohr 51a aus dem äußeren Teleskoprohr 51b herausbewegt und wieder hineinbewegt werden.

Die Längsachse Z der Einstelleinheit 30 ist zugleich die Mittelachse der Antriebsspindel 48 und der Teleskoprohre 51a, 51b, so dass die Längsachse Z gleichzeitig Drehachse Z ist, um die dann der Positionierungskopf 42 zusammen mit der Fußaufnahme 18 mit Hilfe des Rotationsantriebs 53 drehbar ist.

Zwischen dem ersten Handrad 34 und dem zweiten Handrad 36 ist ein Betätigungselement 54 angeordnet, bei dessen Betätigung eine Drehung des äußeren Teleskoprohrs 51b zusammen mit dem inneren Teleskoprohr 51a auch ohne eine Drehbewegung des zweiten Handrades 36 ermöglicht. Zum Betätigen des Betätigungselements 54 wird dieses entlang der Drehachse Z in Richtung des Positionierungskopfs 42 verschoben. Der Betätigungshub entlang der Drehachse Z ist durch den Abstand des vom Positionierungskopf 42 abgewandten Ende der Nabe 37 des zweiten Handrads 36 und einem am Betätigungselement 54 vorgesehenen Vorsprung 54a begrenzt. Um eine vom zweiten Handrad 36 unabhängige Drehung zu ermöglichen, steht das Betätigungselement 54 über drei axial verschiebbare Stifte in Eingriff, von denen ein in Figur 3 sichtbarer Stift mit den Bezugszeichen 56a bezeichnet ist. Die drei Stifte sind in gleichen Winkelabständen um die Drehachse Z angeordnet und sind in den weiteren Figuren auch mit den Bezugszeichen 56a, 56b, 56c bezeichnet.

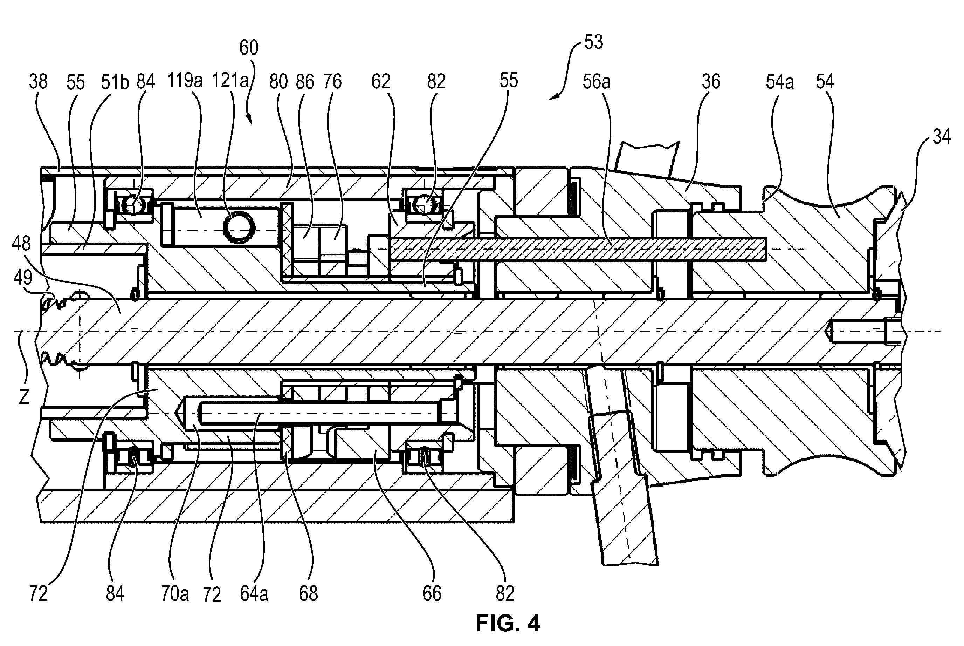

Figur 4 zeigt eine vergrößerte Darstellung eines Ausschnitts des Längsschnitts nach Figur 3 mit Elementen des Rotationsantriebs 53. Der Rotationsantrieb 53 umfasst eine Vorrichtung 60 zum selbsthemmenden bidirektionalen Antrieb der Abtriebswelle 55. Das zweite Handrad 36 steht über die Stifte 56a bis 56c mit einem Rotationskörper 62 in Eingriff, so dass eine Drehbewegung des zweiten Handrads 36 auf den Rotationskörper 62 übertragen wird. Der Rotationskörper 62 ist im Gehäuse 38 über ein erstes Kugellager 82 drehbar um die Drehachse Z aufgenommen. Ferner ist der Rotationskörper 62 frei drehbar auf dem dem zweiten Handrad 36 zugewandten Ende der Abtriebswelle 55 angeordnet. Weiterhin sind sowohl das zweite Handrad 36, das Betätigungselement 54 als auch die Abtriebswelle 55 jeweils frei drehbar auf der Antriebsspindel 48 angeordnet. Ferner ist das dem Positionierungskopf 42 zugewandte Ende der Abtriebswelle 55 über ein zweites Kugellager 84 im Gehäuse 38 aufgenommen. Ferner ragen drei fest mit dem Rotationskörper 62 verbundene Mitnahmestifte, von denen in Figur 4 sichtbarer Mitnahmestift mit dem Bezugszeichen 64a und die weiteren in den weiteren Figuren sichtbaren Mitnahmestifte mit den Bezugszeichen 64b und 64c bezeichnet sind, in drei in einem ein Innenteil 72 des Rotationsantriebs 53 bildenden Bereich der Abtriebswelle 55 vorgesehene Bohrungen, von denen die in Figur 4 sichtbare Bohrung mit dem Bezugszeichen 70a und die weiteren in den weiteren Figuren sichtbaren Bohrungen mit den Bezugszeichen 70b und 70c bezeichnet sind. Die Mitnahmestifte 64a bis 64c sind hierbei durch eine Rotationsfreigabescheibe 66 hindurchgeführt, wobei die Rotationsfreigabescheibe 66 in Richtung der Drehachse Z mithilfe der Stifte 56a bis 56c in Richtung des Positionierungskopfs 42 zur Freigabe der Rotation der Abtriebswelle 55 ohne Betätigung des zweiten Handrads 36 verschiebbar ist.

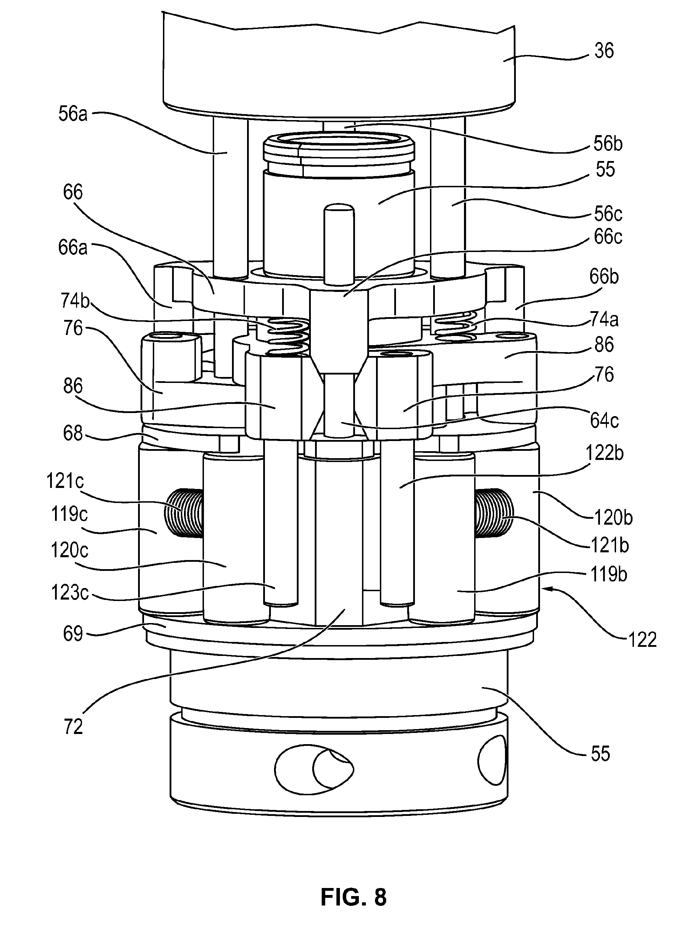

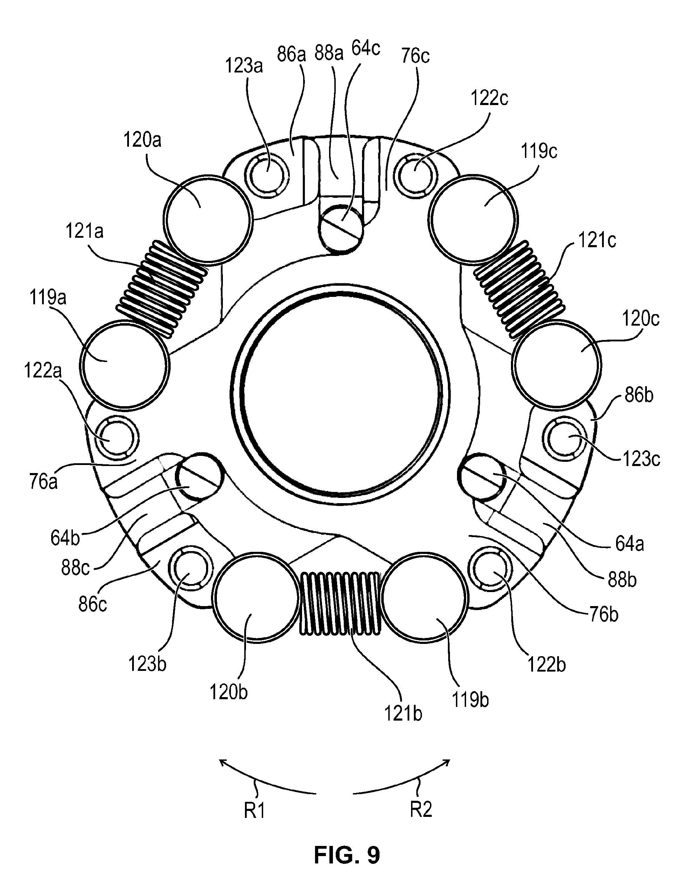

Der Durchmesser der Bohrungen 70a bis 70c ist im vorliegenden Ausführungsbeispiel 2 mm größer als der Durchmesser der Mitnahmestifte 64a bis 64c. Somit sind die Mitnahmestifte 64a bis 64c mit einem Spiel von 2mm, d.h. von 1mm in jede Drehrichtung R1, R2, in den Bohrungen 70a bis 70c aufgenommen, so dass bei einer Drehung des zweiten Handrads 36 im Bereich des Spiels keine Drehung der Abtriebswelle 55 erfolgt. Wie in Figur 9 gut zu sehen ist, sind die Mitnahmestifte 64a bis 64c dabei zwischen den Armen 76a bis 76c eines ersten Rotationssterns 76 und zwischen den Armen 86a bis 86c eines zweiten Rotationssterns 86 hindurchgeführt, mit denen als Freigabeelemente dienende Freigabestifte fest verbunden sind. Die Längsachsen der Freigabestifte 64a bis 64c sowie die Längsachsen der Mitnahmestifte 64a bis 64c verlaufen parallel zur Drehachse Z. Die mit dem jeweiligen Rotationsstern 76, 86 verbundenen Freigabestifte werden bei einer durch die Drehung der Mitnahmestifte 64a bis 64c im Bereich des zwischen den Bohrungen 70a bis 70c und den Mitnahmestiften 64a bis 64c vorhandenen Spiels gedreht, wobei abhängig von der Drehrichtung der erste Rotationsstern 76 oder der zweite Rotationsstern 86 gedreht wird. Bei einer Betätigung des Betätigungselements 54 wird über die Stifte 56a bis 56c die Rotationsfreigabescheibe 66 in Richtung des Innenteils 72 bewegt, wodurch beide Rotationssterne 76, 86 gleichzeitig in entgegengesetzte Drehrichtungen gedreht werden, wie später in Verbindung mit den Figuren 8 und 9 noch näher erläutert wird. Zwischen dem Innenteil 72 und den Rotationssternen 76, 86 ist eine mit Öffnungen zum Durchführen der Mitnahmestifte 64a bis 64c versehene Zwischenscheibe 68 angeordnet.

Der erste Rotationsstern 76 und der zweite Rotationsstern 86 sind auf der Abtriebswelle 55 drehbar um die Drehachse Z gelagert. Um das Innenteil 72 herum ist ein drehfest mit dem Gehäuse 38 verbundenes Außenteil 80 angeordnet, das zumindest im Bereich des Innenteils 72 eine kreisrunde Öffnung 80a hat. Zwischen dem Innenteil 72 und dem Außenteil 80 sind insgesamt sechs Klemmrollen angeordnet, von denen die in Figur 4 sichtbare Klemmrolle mit dem Bezugszeichen 119a bezeichnet ist.

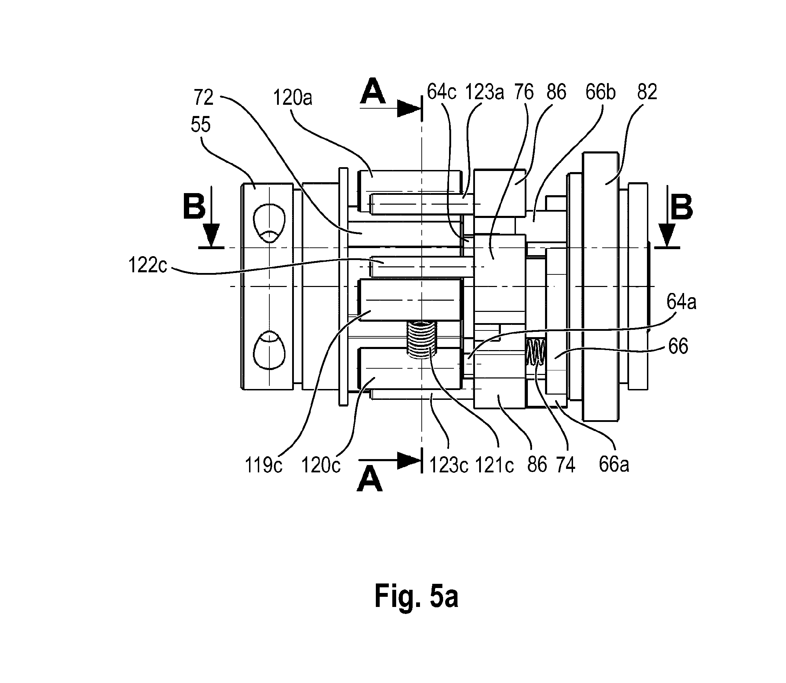

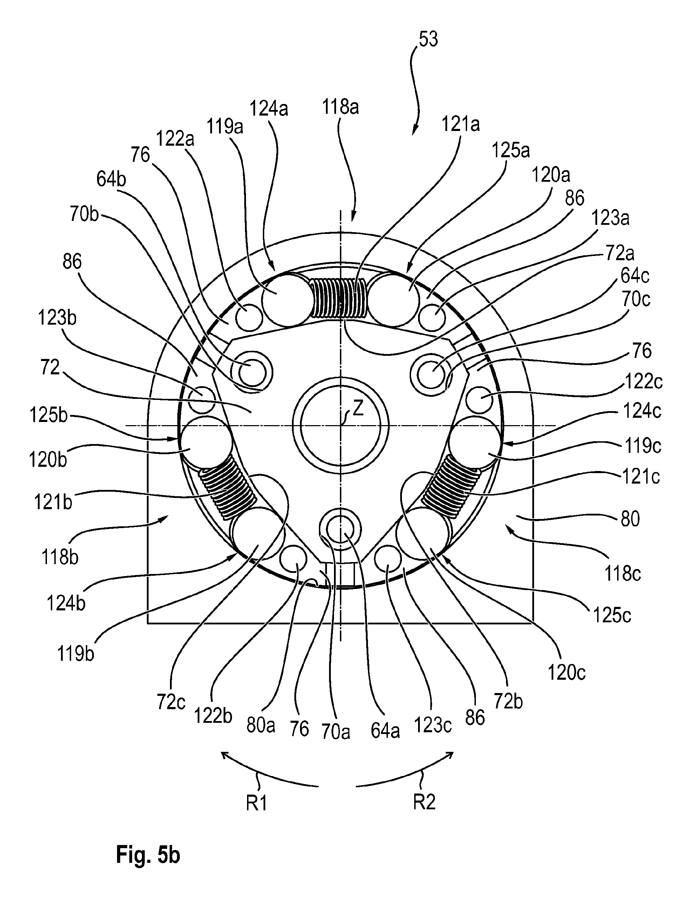

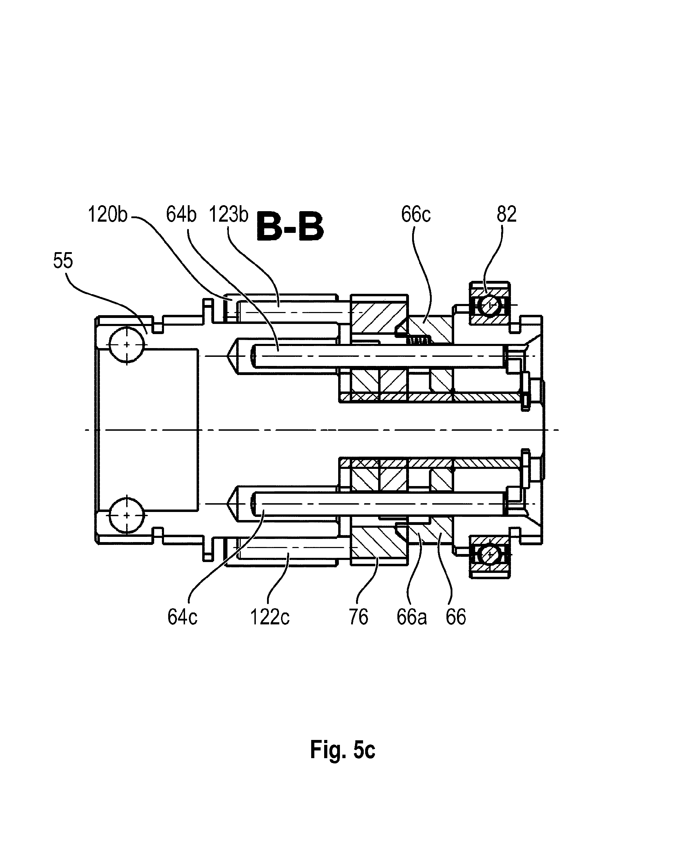

Figur 5a zeigt eine Seitenansicht mit einer Anordnung von ausgewählten Elementen des Rotationsantriebs 53 und Figur 5b einen Schnitt der Anordnung nach Figur 5a entlang der Schnittlinie A-A. Figur 5c zeigt eine Schnittdarstellung der Anordnung nach Figur 5a entlang der Schnittlinie B-B.

Das Innenteil 72 hat eine im Wesentlichen dreieckige Grundform mit gleicher Schenkellänge der Schenkel 72a bis 72c. Ferner ist in Figur 5b das ortsfest im Gehäuse 38 angeordnete Außenteil 80 zusätzlich dargestellt. Zwischen den Schenkeln 72a bis 72c und der inneren kreisrunden Öffnungen 80a des Außenteils 80 sind jeweils in gleichen Winkelabständen um die Drehachse Z Sperr- und Freigabeanordnungen 118a bis 118c angeordnet, wie dies am Besten in Figur 5b zu sehen ist. Die Elemente der einzelnen Sperr- und Freigabeanordnungen 118a bis 118c sind jeweils mit der gleichen Bezugszeichenziffer und einem fortlaufenden Kleinbuchstaben bezeichnet, wobei für die Elemente der ersten Sperr- und Freigabeanordnung 118a der Buchstabe a, für die Elemente der zweiten Sperr- und Freigabeanordnung 118b der Buchstabe b und für die Elemente der dritten Sperr- und Freigabeanordnung 118c der Buchstabe c verwendet wird. Aufgrund des gleichen Aufbaus der Sperr- und Freigabeeinheiten 118a bis 118c wird im Folgenden nur die erste Sperr- und Freigabeeinheit 118a ausführlich beschrieben. Die Erläuterungen zum Aufbau und Funktion der ersten Sperr- und Freigabeeinheit 118a gelten auch für die weiteren Sperr- und Freigabeeinheiten 118b und 118c.

Die Sperr- und Freigabeanordnung 118a umfasst eine zwischen dem Schenkel 72a des Innenteils 72 und der kreisrunden Öffnung 80a des Außenteils 80 angeordnete Klemmrollen 119a, 120a, eine zwischen den Klemmrollen 119a, 120a angeordnete Druckfeder 121a, ein erstes Freigabeelement 122a, das auf der der Feder 121a gegenüberliegenden Seite der Klemmrolle 119a angeordnet ist und ein zweites Freigabeelement 123a, das auf der der Feder 121a gegenüberliegenden Seite der Klemmrolle 120a angeordnet ist. Der Querschnittsbereich zwischen dem Schenkel 72a und der kreisrunden Öffnung 80a verjüngt sich zu dem Schenkelenden des Schenkels 72a hin, so dass die Mantelflächen der Klemmrollen 119a, 120a in der in Figur 5b gezeigten Position sowohl an der Innenseite der kreisrunden Öffnung 80a als auch am äußeren Schenkel 72a anliegen, so dass sie bei einer Drehung des Innenteils 72 in der ersten Drehrichtung R1 die Klemmrolle 119a zwischen dem Schenkel 72a und der kreisrunden Öffnung 80a eingeklemmt wird.

Bei einer Drehung des Innenteils 72 in die entgegengesetzte zweite Drehrichtung R2 wird die Klemmrolle 120a zwischen der kreisrunden Öffnung 80a und dem Schenkel 72a eingeklemmt. Somit wird eine Drehung des Innenteils 72 gegenüber dem ortsfesten Außenteil 80 durch die Klemmrollen 119a, 120a verhindert. Der Bereich zwischen dem Schenkel 72a und der kreisrunden Öffnung 80a, in dem die Rolle 119a angeordnet ist, wird als erster Klemmbereich 124a und der Bereich zwischen dem Schenkel 72a und der kreisrunden Öffnung 80a, in dem die zweite Klemmrolle 120a angeordnet ist, als zweiter Klemmbereich 125a bezeichnet. Das Außenteil 80, das Innenteil 72 und die erste Klemmrolle 118a bilden somit eine erste Sperreinheit und das Außenteil 80, das Innenteil 72 und die zweite Klemmrolle 125a bilden eine zweite Sperreinheit, wobei die erste Sperreinheit mit der Klemmrolle 119a eine Drehung des Innenteils in der Drehrichtung R1 verhindert und die Sperreinheit mit der Klemmrolle 120a eine Drehung des Innenteils 72 in der Drehrichtung R2 verhindert.

Die Klemmrollen 119a, 120a werden mithilfe der Feder 120a in ihren jeweiligen Klemmbereich 124a, 125a gedrückt. Liegt ein abtriebsseitiges Drehmoment an der Abtriebswelle 55 an, das insbesondere über die Fußaufnahme 18 in die Einstelleinheit 30 eingeleitet wird und von dem Teleskoprohren 51a, 51b auf die Abtriebswelle 55 übertragen wird, sperren die Sperr- und Freigabeanordnungen 118a bis 118c eine Drehbewegung, so dass eine Rotation der Fußaufnahme 18 sicher verhindert wird.

Soll jedoch die Rotation der Fußaufnahme 18 aktiv verstellt werden, so wird das zweite Handrad 36 gedreht, so dass über den Rotationskörper 62 eine Drehung der Mitnahmestifte 64a bis 64c um die Drehachse Z herum erfolgt. Da die Mitnahmestifte 64a bis 64c, wie bereits erwähnt, in der jeweiligen Bohrung 70a bis 70c mit Spiel aufgenommen sind, erfolgt eine Drehung der Mitnahmestifte 64a bis 64c in einem ersten Winkelbereich um die Drehachse Z herum, ohne dass das Innenteil 72 mitgedreht wird. Bei einer Drehung der Mitnahmestifte 64a bis 64c in der Drehrichtung R1 wird der erste Rotationsstern 76 mit den Freigabestiften 122a, 122b und 122c in Drehrichtung R1 gedreht, so dass der Freigabestift 122a von einer Neutralpostition in eine Freigabeposition bewegt wird und dabei die Klemmrolle 119a kontaktiert und aus ihrem Klemmbereich 124a drückt. Dadurch kann die Klemmrolle 119a die Drehung des Innenteils 72 nicht mehr sperren. Die Bewegung des Innenteils 72 in Drehrichtung R1 ist somit freigegeben, so dass bei einer weiteren Drehung der Mitnahmestifte 64a bis 64c in Drehrichtung R1 diese die Wandungen der Bohrungen 70a bis 70c kontaktieren und das Innenteil 72 zusammen mit der Antriebswelle 55 drehen. Im Unterschied zum Innenteil 72 steht Rotationsstern 76 somit in Drehrichtung R1 spielfrei mit den Mitnahmestiften 64a bis 64c in Eingriff oder mit wesentlich geringerem Spiel in Eingriff, als die Mitnahmestifte 64a bis 64c und die Bohrungen 70a bis 70c in Eingriff stehen.

Bei der Drehung des Innenteils 72 wird die einteilig oder einstückig mit dem Innenteil 72 ausgebildete Abtriebswelle 55 gedreht, so dass die Teleskoprohre 51a, 51b und der am vorderen Ende des inneren Teleskoprohrs 51a angeordnete Positionierungskopf 42 zusammen mit dem Innenteil 72 gedreht werden. Wird keine Antriebskraft mehr über das Handrad 36 ausgeübt, wird die Klemmrolle 119a durch die Federkraft der Feder 121a zurück in den Klemmbereich 124a gedrückt, wodurch das Freigabeelement 122a zurück in seine Neutralposition bewegt wird, so dass eine Drehung des Innenteils 72 in Drehrichtung R1 durch die Klemmrolle 119a nachfolgend sicher vermieden wird, auch dann, wenn über die Fußaufnahme 10 ein Drehmoment zur Drehung des Innenteils 72 in Drehrichtung R1 übertragen wird. Beim Zurückbewegen des Freigabeelements 122a wird der Rotationsstern 76 in die zweite Drehrichtung R2 gedreht, so dass alle mit dem Rotationsstern 76 verbundene Freigabeelemente 122a bis 122c zurück in ihre Neutralposition bewegt werden.

Bei einer Drehung des zweiten Handrads 36 in Drehrichtung R2 wird die Drehbewegung im Wesentlichen spielfrei über den Rotationskörper 62 auf die Mitnahmestifte 64a bis 64c übertragen, die in Drehrichtung R2 um die Drehachse Z herum gedreht werden. Wie bereits erwähnt, sind die Mitnahmestifte 64a bis 64c mit Spiel in den Bohrungen 70a bis 70c des Innenteils 72 aufgenommen, so dass die Mitnahmestifte 64a bis 64c einen Winkelbetrag in Drehrichtung R2 gedreht werden können, bis sie an den Wandungen der Bohrungen 70a bis 70c anstoßen und ein Antriebsdrehmoment auf das Innenteil 72 ausüben. Bei dieser Winkelverstellung innerhalb des Spiels drehen die Mitnahmestifte 64a bis 64c um den zweiten Rotationsstern 86 in Drehrichtung R2, so dass das Freigabeelement 123a die Klemmrolle 120a kontaktiert und gegen die Federkraft der Feder 121a aus dem Klemmbereich 125a drückt, so dass das Innenteil 72 anschießend in Drehrichtung R2 gedreht werden kann, ohne dass die Klemmrolle 120a in dem Zwischenraum zwischen dem Schenkel 72a und der kreisrunden Öffnung 80a des Außenteils 80 eingeklemmt wird. Dadurch ist eine Drehung des Innenteils 72 und somit der Abtriebswelle 55 und der mit der Abtriebswelle 55 verbundenen Teleskoprohranordnung 55 in Drehrichtung R2 durch bloße Betätigung des zweiten Handrades 36 möglich. Wird das zweite Handrad 36 nicht mehr in Drehrichtung R2 gedreht bzw. wird es losgelassen, drückt die Feder 121a die Klemmrolle 120a in den Klemmbereich 125a zurück, wodurch die Klemmrolle 120a das Freigabeelement 123a von der Freigabeposition wieder zurück in seine Neutralposition drückt, wobei der gesamte Rotationsstern 86 in Drehrichtung R1 gedreht wird, bis alle Elemente die in Figur 5b gezeigte Position haben. In dieser Position wird eine Drehung durch ein auf der Abtriebswelle 55 wirkenden Drehmoments wieder durch die Sperr- und Freigabeeinheiten 118a bis 118c einfach verhindert. Eine Drehung ist somit in dem beschriebenen Rotationseinstellmodus des Rotationsantriebs 53 nur über eine Drehung des zweiten Handrads 36 möglich. Der Rotationsantrieb 53 ist, wie nachfolgend in Verbindung mit den Figuren 8 und 9 noch ausführlich beschrieben wird, auch in einem Rotationsfreigabemodus betreibbar, der mithilfe des bereits erwähnten Betätigungselements 54 aktivierbar ist. In diesem Rotationsfreigabemodus werden mithilfe der Freigabeelemente 122a, 123a beide Klemmrollen 119a, 120 gleichzeitig aus ihren Klemmbereichen 124a, 125a bewegt, so dass eine Drehbewegung des Innenteils 72 freigegeben ist und das Innenteil 72 und alle drehfest mit dem Innenteil 72 verbundenen Elemente 55, 51a, 51b, 42, 44, 18 sowohl über das zweite Handrad 36 als auch über ein auf die Abtriebswelle 55, insbesondere über die Fußaufnahme 18, wirkendes Drehmoment frei in beide Drehrichtungen R1 und R2 gedreht werden können.

Hierzu steht das Betätigungselement 54 über die axial verschiebbaren Stifte 56a bis 56c mit der Rotationsfreigabescheibe 66 in Eingriff (siehe Stift 56a in den Figuren 5a, 5c) und drücken die Rotationsfreigabescheibe 66 zum Innenteil 72 hin, wodurch die Rotationsfreigabescheibe 66 gleichzeitig den ersten Rotationsstern 76 in Drehrichtung R1 und den zweiten Rotationsstern 86 in Drehrichtung R2 dreht, wodurch die mit dem Rotationssternen 76, 86 verbundenen Freigabestifte 122a bis 122c, 123a bis 123c gleichzeitig von ihren Neutralpositionen in ihre Freigabepositionen bewegt und dadurch die Klemmrollen 119a bis 119c und 120a bis 120c gleichzeitig aus ihren Klemmbereichen 124a bis 124c, 125a bis 125c drücken. Über eine zwischen den Rotationssternen 76, 86 und der Rotationsfreigabescheibe 66 angeordnete Federn 74a, 74b und74c, von denen in Figur 5a nur die Feder 74a sichtbar ist, wird eine Rückstellkraft zum Zurückstellen des Betätigungselements 54 in die in Figur 4 gezeigte Position bewirkt, so dass dann, wenn das Betätigungselement 54 nicht mehr betätigt ist, die Rotationsfreigabescheibe 66 und das über die Stifte 56a bis 56c verbundene Betätigungselement 54 zurückbewegt wird und eine Rückstellung der Freigabeelemente 122a bis 122c, 123a bis 123c durch die Federkraft der Federn 121a bis 121c erfolgt. Dadurch werden die Klemmrollen 119a bis 119c, 120a bis 120c automatisch zurück in ihre Klemmbereiche 124a bis 124c, 125a bis 125c bewegt, so dass eine Drehung des Innenteils 72 durch ein abtriebsseitiges Drehmoment wieder einfach und sicher durch die Sperr- und Freigabeanordnungen 118a bis 118c verhindert wird.

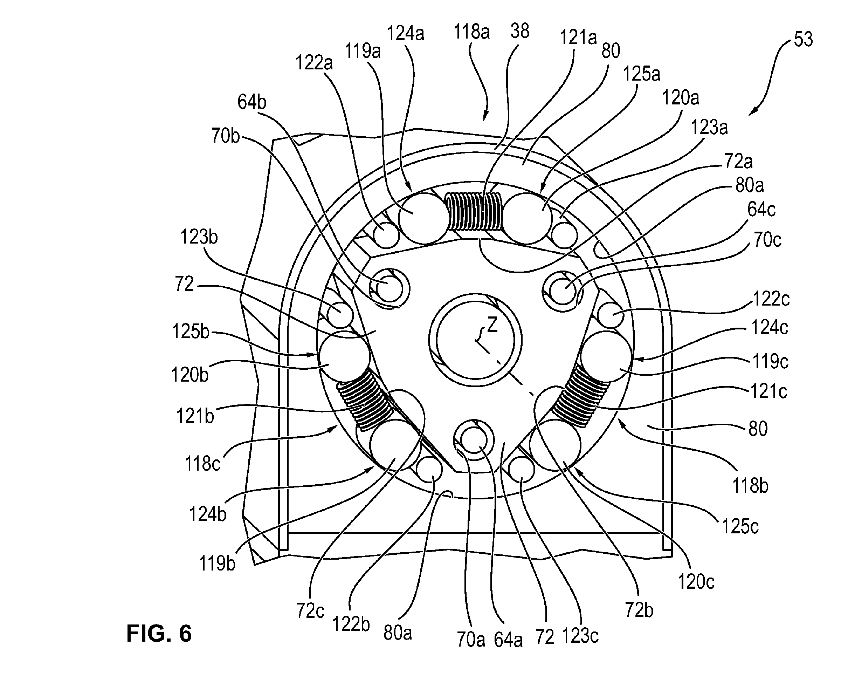

Figur 6 zeigt eine perspektivische Darstellung eines Querschnitts des Rotationsantriebs 53 und durch das Gehäuse 38 entlang der Schnittlinie A-A nach Figur 5a.

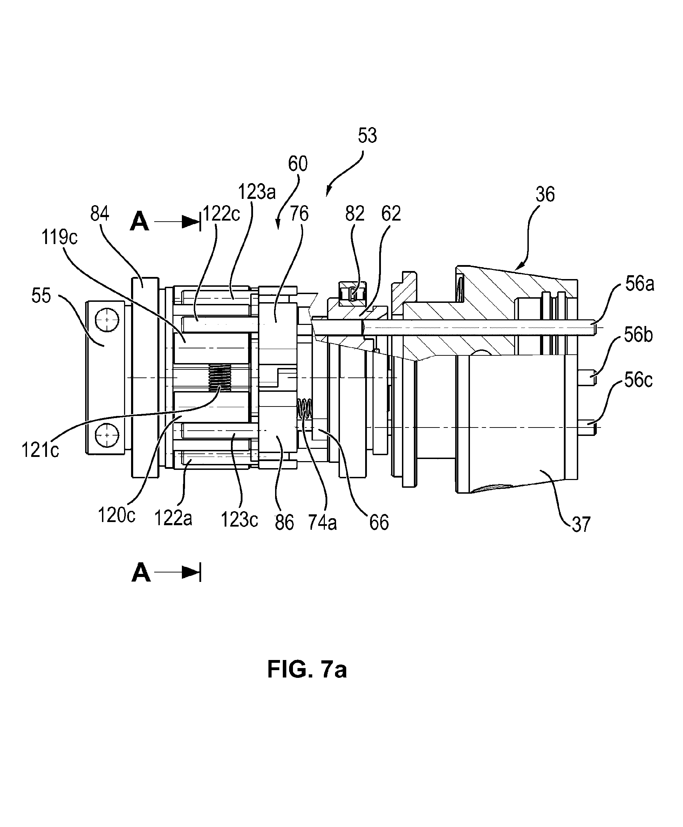

In Figur 7a ist eine weitere Ansicht von Elementen des Rotationsantriebs 53 in einer teilgeschnittenen Darstellung, ähnlich der nach Figur 5a, wobei die Elemente des Rotationsantriebs 53 gegenüber Figur 5a in einer anderen Winkelstellung dargestellt sind. Ferner sind in Figur 7a zusätzlich die Nabe 37 des zweiten Handrads 36 ohne die drei zum Drehen der Nabe 37 in diese einschraubbare Hebel dargestellt.

Die drei Stifte 56a bis 56c ragen durch die Nabe 37 hindurch und erstrecken sich weiter durch den Rotationskörper 62 hindurch. Dadurch übertragen die Stifte 56a bis 56c eine Drehbewegung des Handrads 36 auf den Rotationskörper 62. Die Stifte 56a bis 56c sind in gleichen Winkelabständen um die Drehachse Z angeordnet. Die mit dem Rotationskörper 62 fest verbundenen Mitnahmestifte 64a bis 64c sind auf einer anderen Kreisbahn oder versetzt zu den Stiften 56a bis 56c in gleichen Winkelabständen um die Drehachse Z herum angeordnet. Somit wird die von dem zweiten Handrad 36 über die Stifte 56a bis 56c auf den Rotationskörper 62 übertragene Drehbewegung ohne Spiel auf die Mitnahmestifte 64a bis 64c übertragen, die dann wie beschrieben die Position der Freigabeelemente 123a bis 123c, 124a bis 124c abhängig von der Drehrichtung R1, R2 aus einer Neutralstellung in eine Freigabestellung bewegen und anschließend das Innenteil 72 drehen. Wie bereits erwähnt, dienen die Stifte 56a bis 56c auch zur Kopplung des Betätigungselements 54 mit der Rotationsfreigabescheibe 66, indem die Stifte 56a bis 56c durch das Betätigungselement 54 in axialer Richtung zu den Rotationssternen 76, 86 hin verschoben werden und die Rotationsfreigabescheibe 66 in Richtung der Rotationssterne 76, 86 verschiebt.

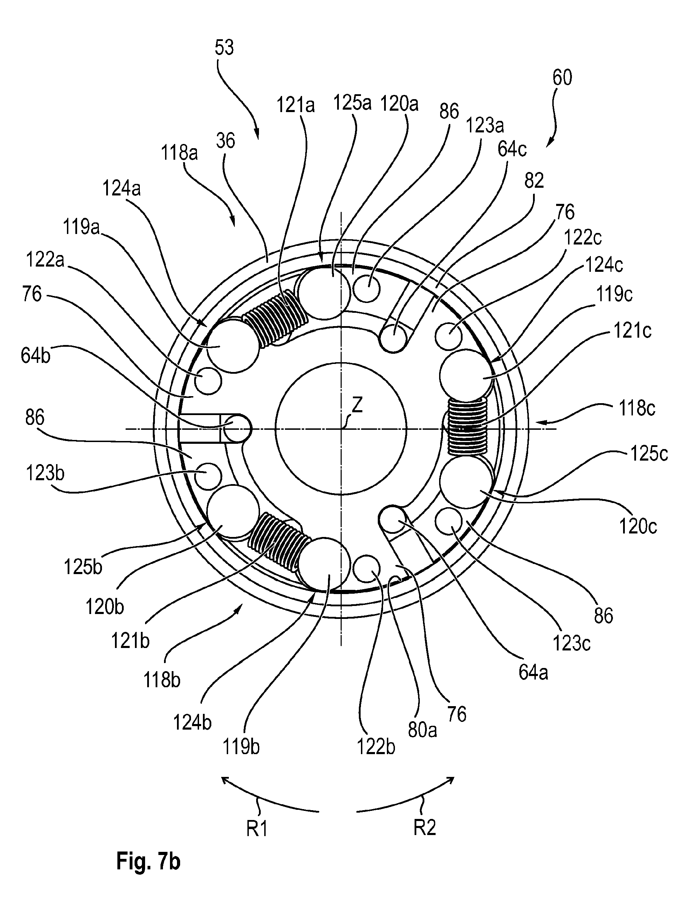

In Figur 7b ist eine Schnittdarstellung der in Figur 7a gezeigten Anordnung mit Elementen des Rotationsantriebs 53 entlang der Schnittlinie A-A nach Figur 7a dargestellt. Sowohl in Figur 7a als auch in Figur 7b sind weder das Außenteil 80 noch das Gehäuse 38 dargestellt.

Figur 8 zeigt eine weitere perspektivische Darstellung einer Anordnung mit Elementen des Rotationsantriebs 53, in der die Elemente zur Freigabe einer von der Betätigung des zweiten Handrads 36 unabhängigen Drehbewegung der Abtriebswelle 55 gut sichtbar sind. Wie bereits zuvor erläutert, werden die Stifte 56a bis 56c bei einer Betätigung des Betätigungselements 54 in Richtung der Rotationssterne 76, 86 verschoben und drücken dabei die Rotationsfreigabescheibe 66 in Richtung der Rotationssterne 76, 86. Die Rotationsfreigabescheibe 66 hat dazu in Richtung der Rotationssterne 76, 86 vorstehende Rotationsfreigabeelemente 66a bis 66c, die zwischen Arme 86a bis 86c und 76a bis 76c der Rotationssterne 76, 86 gebildete Bereiche 88a bis 88c z gedrückt werden und dadurch die Rotationssterne 76, 86 gegeneinander verdrehen, so dass alle Freigabeelemente 122a bis 122c, 123a bis 123c gleichzeitig aus ihrer Neutralstellung in ihre Freigabestellung bewegt werden. Dadurch werden alle Klemmrollen 119a bis 119c, 120a bis 120c aus ihren Klemmbereichen 124a bis 124c, 125a bis 125c bewegt, so dass eine freie Drehung der Abtriebswelle auch durch abtriebsseitige Drehmomente möglich ist. Wird das Betätigungselement 54 zurück in seine Ausgangslage bewegt, drücken die Stifte 56a bis 56c die Rotationsfreigabescheibe 66 nicht mehr in Richtung der Rotationssterne 76, 86, so dass die Rotationsfreigabescheibe 66 durch die Federn 74a bis 74c entlang der Drehachse Z zurück in Richtung der Antriebsräder 34, 36 bewegt wird, so dass die Rotationsfreigabeelemente 66a bis 66c nicht mehr zwischen die Arme 76a bis 76c, 86a bis 86c der Rotationssterne 76, 86 gedrückt sind und die Klemmrollen 119a bis 119c, 120a bis 120c durch die auf sie wirkende Federkraft der Federn 121a bis 121c zurück in die Klemmbereiche 124a bis 124c, 125a bis 125c, gedrückt und alle Freigabeelemente 122a bis 122c, 123a bis 123c aus ihrer Freigabeposition in ihre Neutralposition bewegt werden.

Alternativ zum beschriebenen Betätigen des Betätigungselements 54 durch Drücken des Betätigungselements in Richtung des zweiten Handrads bzw. in Richtung des vorderen Endes der Einstelleinheit 30 kann bei einer anderen Ausführung das Betätigen des Betätigungselements 54 auch durch Ziehen des Betätigungselements in Richtung des ersten Handrads 36 bzw. in Richtung des hinteren Endes der Einstelleinheit 30 erfolgen. Insbesondere wird die Rotationsfreigabescheibe 66 gewendet, so dass die Rotationsfreigabeelemente 66a bis 66c zum Betätigungselement 54 hin hervorstehen. Ferner wird die Rotationsfreigabescheibe 66 dann zwischen dem Innenteil 72 und den Rotationssternen 76, 86 angeordnet.

### Bezugszeichenliste

- 10: Operationstisch
- 12: Operationstischfuß
- 14: Operationstischsäule
- 16: Patientenlagerfläche
- 17: Extensionsset
- 18: erste Fußaufnahme
- 20: zweite Fußaufnahme
- 22: erste Verbindungseinheit
- 24: zweite Verbindungseinheit
- 26: erster Extensionsholm
- 28: zweiter Extensionsholm
- 30: Einstelleinheit zur Rotations- und Längenverstellung
- 32: Verbindungsstab
- 34: erstes Handrad
- 36: zweites Handrad
- 38: Gehäuse
- 40: Gelenk
- 41: Gelenkkopfaufnahme
- 42: Positionierungskopf
- 44: Verbindungselement
- 46: Klemmverschluss
- 48: Antriebsspindel
- 49: Außengewinde
- 50: Lager
- 51: Teleskoprohranordnung
- 51a: inneres Teleskoprohr
- 51b: äußeres Teleskoprohr
- 52: Schlitten
- 52a: Innengewinde
- 52b, 52c: Nase
- 52d: Indikatorring
- 52e: Skala
- 53: Rotationsantrieb
- 54: Betätigungselement
- 54a: Vorsprung
- 55: Abtriebswelle
- 56a, 56b, 56c: Stift
- 60: Vorrichtung zum selbsthemmenden bidirektionalen Antrieb
- 62: Rotationskörper
- 64a, 64b, 64c: Mitnahmestifte
- 66: Rotationsfreigabescheibe
- 66a, 66b, 66c: Rotationsfreigabeelemente
- 68: Zwischenscheibe
- 70a, 70b, 70c: Bohrungen
- 72: Innenteil
- 74a, 74b, 74c: Feder
- 76: erster Rotationsstern
- 76a, 76b, 76c: Arme des zweiten Rotationssterns
- 80: Außenteil
- 80a: kreisförmige Öffnung
- 82, 84: Kugellager
- 86: zweiter Rotationsstern
- 86a, 86b, 86c: Arme des zweiten Rotationssterns
- 88a, 88b, 88c: Bereiche
- 118a, 118b, 118c: Sperr- und Freigabeanordnungen
- 119a, 119b, 119c 120a, 120b, 120c: Klemmrollen
- 121a, 121b, 121c: Feder
- 122a, 122b, 122c 123a, 123b, 123c: Freigabeelemente
- 124a, 124b, 124c, 125a, 125b, 125c: Klemmbereich
- Z: Drehachse
- R1: erste Drehrichtung
- R2: zweite Drehrichtung

## Patentansprüche

1. Vorrichtung zum selbsthemmenden bidirektionalen Antrieb einer medizinischen Behandlungsvorrichtung (18),
mit einem um eine Drehachse (Z) drehbaren Innenteil (72),
einem rotationsfesten Außenteil (80) mit einer kreisförmigen Öffnung (80a), die das Innenteil (72) umgibt und konzentrisch um die Drehachse (Z) angeordnet ist,
einer um die Drehachse (Z) drehbaren Abtriebswelle (55), die mit dem Innenteil (72) verbunden und mit der Behandlungsvorrichtung (18) verbindbar ist,
einem Antriebselement (36) zum Drehen des Innenteils (72) zusammen mit der Abtriebswelle (55) um die Drehachse (Z),
mit einem ersten Klemmkörper (119a bis 119c) und mit einem zweiten Klemmkörper (120a bis 120c), die in einem Zwischenraum zwischen dem Innenteil (72) und dem Außenteil (80) angeordnet sind, **dadurch gekennzeichnet,**
**dass** das Außenteil (80), das Innenteil (72) und der erste Klemmkörper (119a bis 119c) eine erste Sperreinheit zum Sperren der Drehung des Innenteils (72) in einer ersten Drehrichtung (R1) und zum Freigeben der Drehung des Innenteils (72) in einer zweiten Drehrichtung (R2) bilden,
**dass** das Außenteil (80), das Innenteil (72) und der zweite Klemmkörper (120a bis 120c) eine zweite Sperreinheit zum Sperren der Drehung des Innenteils (72) in der zweiten Drehrichtung (R2) und zum Freigeben der Drehung des Innenteils (72) in der ersten Drehrichtung (R1) bilden,
**dass** ein durch das Antriebselement (36) in eine erste Freigabeposition bewegbares erstes Freigabeelement (122a bis 122c) vorgesehen ist, das das Sperren der Drehung des Innenteils (72) in der ersten Drehrichtung (R1) durch die erste Sperreinheit verhindert, und
**dass** ein durch das Antriebselement (36) in eine zweite Freigabeposition bewegbares zweites Freigabeelement (123a bis 123c) vorgesehen ist, das das Sperren der Drehung des Innenteils (72) in der zweiten Drehrichtung (R2) durch die zweite Sperreinheit verhindert.

2. Vorrichtung nach Anspruch 1, **dadurch gekennzeichnet, dass** das Antriebselement (36) bei einer Drehung in die erste Drehrichtung (R1) zuerst das erste Freigabeelement (122a bis 122c) aus einer ersten Neutralposition in die erste Freigabeposition bewegt, in der das erste Freigabeelement (122a bis 122c) das Sperren der Drehung des Innenteils (72) in der ersten Drehrichtung (R1) durch die erste Sperreinheit verhindert, und bei einer weiteren Drehung das Innenteil (72) in die erste Drehrichtung (R1) dreht, und
dass das Antriebselement (36) bei einer Drehung in die zweite Drehrichtung (R2) zuerst das zweite Freigabeelement (123a bis 123c) aus einer zweiten Neutralposition in die zweite Freigabeposition bewegt, in der das zweite Freigabeelement (123a bis 123c) das Sperren der Drehung des Innenteils (72) in der zweiten Drehrichtung (R2) durch die erste Sperreinheit verhindert, und bei einer weiteren Drehung das Innenteil (72) in die zweite Drehrichtung (R2) dreht.

3. Vorrichtung nach einem der Ansprüche 1 oder 2, **dadurch gekennzeichnet, dass** der erste Klemmkörper (119a bis 119c) in einem zwischen dem Außenteil (80) und dem Innenteil (72) gebildeten ersten Klemmbereich (124a bis 124c) bei einer Drehung des Innenteils (72) in die erste Drehrichtung (R1) einklemmbar ist, und
dass der zweite Klemmkörper (120a bis 120c) in einem zwischen dem Außenteil (80) und dem Innenteil (72) gebildeten zweiten Klemmbereich (125a bis 125c) einklemmbar ist.

4. Vorrichtung nach einem der vorhergehenden Ansprüche, **dadurch gekennzeichnet, dass** ein elastisch verformbares Element (121a bis 121c) zwischen dem ersten Klemmkörper (119a bis 119c) und dem zweiten Klemmkörper (120a bis 120c) angeordnet ist, das den ersten Klemmkörper (119a bis 119c) in den ersten Klemmbereich (124a bis 124c) und den zweiten Klemmkörper (120a bis 120c) in den zweiten Klemmbereich (125a bis 125c) drückt.

5. Vorrichtung nach den Ansprüchen 3 und 4, **dadurch gekennzeichnet, dass** das elastische Element eine Feder, vorzugsweise eine zwischen den Klemmkörpern (119a bis 119c, 120a bis 120c) angeordnete Spiralfeder (121a bis 121c), ist.

6. Vorrichtung nach einem der Ansprüche 4 und 5, **dadurch gekennzeichnet, dass** das erste Freigabeelement (122a bis 122c) auf der dem elastisch verformbaren Element (121a bis 121c) abgewandten Seite des ersten Klemmkörpers (119a bis 119c) und das zweite Freigabeelement (123a bis 123c) auf der dem elastisch verformbaren Element (121a bis 121c) abgewandten Seite des zweiten Klemmkörpers (120a bis 120c) angeordnet sind.

7. Vorrichtung nach einem der vorhergehenden Ansprüche, **dadurch gekennzeichnet, dass** die erste Sperreinheit und die zweite Sperreinheit bei einem an der Abtriebswelle (55) und/oder an dem Innenteil (72) anliegenden abtriebsseitigen Drehmoment eine Drehung des Innenteils (72) und der Abtriebswelle (55) ohne Betätigen des Antriebselements (36) verhindern.

8. Vorrichtung nach einem der vorhergehenden Ansprüche, **dadurch gekennzeichnet, dass** die Vorrichtung eine Drehung der Abtriebswelle (55) ohne separate Rotationsfreigabe nur durch Betätigen des Antriebselements (36) ermöglicht.

9. Vorrichtung nach einem der Ansprüche 2 bis 8, **dadurch gekennzeichnet, dass** das Antriebselement (36) über mindestens ein Eingriffselement (64a bis 64c) mit dem Innenteil (72) in Eingriff steht, wobei das Eingriffselement (64a bis 64c) in einer Aussparung (70a bis 70c) des Innenteils (72) mit Spiel aufgenommen ist und/oder wobei das Eingriffselement (64a bis 64c) in einer Aussparung des Antriebselements (36) mit Spiel aufgenommen ist, wobei das Spiel vorzugsweise einen Wert im Bereich von 0,5 mm bis 5 mm hat.

10. Vorrichtung nach Anspruch 9, **dadurch gekennzeichnet, dass** das Eingriffselement ein Stift (64a bis 64c) und die Aussparung eine Bohrung (70a bis 70c) ist, wobei der Stift (64a bis 64c) in die Bohrung (70a bis 70c) ragt und der Durchmesser der Bohrung (70a bis 70c) vorzugsweise um einen Wert im Bereich von 0,5 mm bis 5 mm größer als der Durchmesser des Stifts (64a bis 64c) ist.

11. Vorrichtung nach einem der vorhergehenden Ansprüche, **dadurch gekennzeichnet, dass** das erste Freigabeelement (122a bis 122c) und das zweite Freigabeelement (123a bis 123c) mit einem Betätigungselement (54) in Wirkverbindung stehen, und
dass das Betätigungselement (54) bei einer Betätigung das erste Freigabeelement (122a bis 122c) in die erste Freigabeposition und das zweite Freigabeelement (123a bis 123c) in die zweite Freigabeposition bewegt.

12. Vorrichtung nach einem der vorhergehenden Ansprüche, **dadurch gekennzeichnet, dass** eine erste Sperr- und Freigabeanordnung (118a) zumindest die erste Sperreinheit, die zweite Sperreinheit, das erste Freigabeelement (122a) und das zweite Freigabeelement (123a) umfasst,
dass die Vorrichtung mindestens eine zweite Sperr- und Freigabeanordnung (118b) umfasst, wobei der Aufbau und die Funktion der zweiten Sperr- und Freigabeanordnung (118b) mit dem Aufbau und der Funktion der ersten Sperr- und Freigabeanordnung (118a) übereinstimmen,
dass die Sperr- und Freigabeanordnungen (118a, 118b) in gleichen Winkelabständen um die Drehachse (Z) herum angeordnet sind.

13. Vorrichtung nach Anspruch 12, **dadurch gekennzeichnet, dass** die Vorrichtung drei Sperr- und Freigabeanordnungen (118a bis 118c) umfasst, deren Aufbau und Funktion übereinstimmen und die in gleichen Winkelabständen um die Drehachse (Z) herum angeordnet sind.

14. Vorrichtung nach einem der Ansprüche 12 oder 13, **dadurch gekennzeichnet, dass** die erste Sperr- und Freigabeanordnung (118a) zumindest das erste Freigabeelement (122a), das zweite Freigabeelement (123a), den ersten Klemmkörper (119a) und den zweiten Klemmkörper (120a) umfasst,
dass die zweite Sperr- und Freigabeanordnung (118b) zumindest ein drittes Freigabeelement (122b), ein viertes Freigabeelement (123b), einen dritten Klemmkörper (119b) und einen vierten Klemmkörper (120b) umfasst,
dass die dritte Sperr- und Freigabeanordnung (118c) ein zumindest fünftes Freigabeelement (122c), ein sechstes Freigabeelement (123c), einen fünften Klemmkörper (119c) und einen sechsten Klemmkörper (120c) umfasst,
dass das erste, dritte und fünfte Freigabeelement (122a bis 122c) derart miteinander verbunden sind, dass sie gemeinsam um die Drehachse (Z) bewegbar sind, und
dass das zweite, vierte und sechste Freigabeelement (123a bis 123c) derart miteinander verbunden sind, dass sie gemeinsam um die Drehachse (Z) bewegbar sind,
wobei das erste, dritte und fünfte Freigabeelement (122a bis 122c) gemeinsam relativ zum zweiten, vierten und sechsten Freigabeelement (123a bis 123c) bewegbar sind, vorzugsweise um einen Winkel im Bereich von 0,5° bis 5°.

15. Vorrichtung nach Anspruch 14, **dadurch gekennzeichnet, dass** das Betätigungselement (54) beim Betätigen gleichzeitig das erste, dritte und fünfte Freigabeelement (122a bis 122c) gemeinsam in die erste Drehrichtung (R1) und das zweite, vierte und sechste Freigabeelement (123a bis 123c) in die zweite Drehrichtung (R2) bewegt,
so dass das erste Freigabeelement (122a) den ersten Klemmkörper (119a) kontaktiert und ein Klemmen des ersten Klemmkörpers (119a) im ersten Klemmbereich (124a) verhindert,
so dass das zweite Freigabeelement (123a) den zweiten Klemmkörper (120a) kontaktiert und ein Klemmen des zweiten Klemmkörpers (120a) im zweiten Klemmbereich (125a) verhindert,
so dass das dritte Freigabeelement (122b) einen dritten Klemmkörper (119b) der zweiten Sprerr- und Freigabeanordnung (118b) kontaktiert und ein Klemmen des dritten Klemmkörpers (119b) in einem zwischen dem Innenteil (72) und dem Außenteil (80) gebildeten dritten Klemmbereich (124b) verhindert,
so dass das vierte Freigabeelement (123b) einen vierten Klemmkörper (120b) der zweiten Sprerr- und Freigabeanordnung (118b) kontaktiert und ein Klemmen des vierten Klemmkörpers (120b) in einem zwischen dem Innenteil (72) und dem Außenteil (80) gebildeten vierten Klemmbereich (125b) verhindert,
so dass das fünfte Freigabeelement (122c) einen fünften Klemmkörper (119c) der dritten Sprerr- und Freigabeanordnung (118c) kontaktiert und ein Klemmen des fünften Klemmkörpers (122c) in einem zwischen dem Innenteil (72) und dem Außenteil (80) gebildeten fünften Klemmbereich (124c) verhindert, und/oder
so dass das sechste Freigabeelement (123c) einen sechsten Klemmkörper (120c) der dritten Sprerr- und Freigabeanordnung (118c) kontaktiert und ein Klemmen des sechsten Klemmkörpers (120c) in einem zwischen dem Innenteil (72) und dem Außenteil (80) gebildeten sechsten Klemmbereich (125c) verhindert.
